(19) Europäisches Patentamt
European Patent Office
Office européen des brevets

(11) Numéro de publication: **0 117 176**
**A1**

(12) **DEMANDE DE BREVET EUROPEEN**

(21) Numéro de dépôt: **84400141.2**

(22) Date de dépôt: **23.01.84**

(51) Int. Cl.³: **C 07 D 277/06**
A 61 K 7/06, A 61 K 7/48
A 61 K 31/425

(30) Priorité: **14.02.83 FR 8392459**

(43) Date de publication de la demande:
**29.08.84 Bulletin 84/35**

(84) Etats contractants désignés:
**AT BE CH DE GB IT LI LU NL SE**

(71) Demandeur: **PIERRE FABRE S.A.**
**125, rue de la Faisanderie**
**F-75116 Paris(FR)**

(72) Inventeur: **Dubois, Jacques**
**10, avenue Emile de Villeneuve**
**F-81100 Castres(FR)**

(72) Inventeur: **Cousse, Henri**
**La Foun de los Nobios Chemin de Lastinos**
**F-81100 Castres(FR)**

(72) Inventeur: **Mouzin, Gilbert**
**21, rue Sainte-Foy**
**F-81100 Castres(FR)**

(74) Mandataire: **Doat, Jean-Pierre**
**PIERRE FABRE S.A. Service Propriété Industrielle 17, Avenue Jean Moulin**
**F-81106 Castres Cédex(FR)**

(54) **Le L- thiazolidine-4 carboxylate de zinc, sa préparation, les compositions topiques le contenant et son utilisation.**

(57) L'invention concerne le L-thiazolidine-4 carboxylate de zinc, de formule :

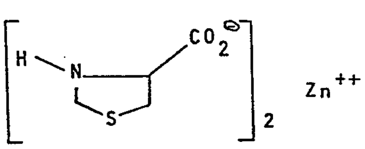

Ce dérivé est utile en dermatologie et en cosmétologie.

EP 0 117 176 A1

Croydon Printing Company Ltd.

La présente invention, réalisée au Centre de Recherches Pierre Fabre, concerne un nouveau dérivé de l'acide L-thiazolidine-4 carboxylique, son procédé de préparation et son application dans le domaine thérapeutique et cosmétologique.

Ce nouveau dérivé est le L-thiazolidine-4 carboxylate de zinc, de formule :

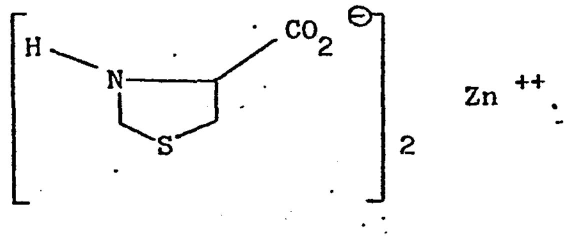

qui possède des propriétés physicochimiques et biologiques permettant son utilisation dans des préparations topiques dermatologiques et cosmétologiques.

Il s'est avéré efficace dans le traitement des divers troubles de la peau et du cuir chevelu, tels que les dermites séborrhéiques, l'acné, les parakératoses, certains érythèmes, la desquamation grasse du cuir chevelu (pellicules) et les alopécies.

Selon la présente invention, l'acide L-thiazolidine-4 carboxylique est associé au zinc sous la forme d'un sel : ces deux constituants associent leurs effets complémentaires favorables pour l'application dermatologique et cosmétologique.

L'acide L-thiazolidine-4 carboxylique est capable, par application percutanée, d'activer les cellules de la racine du cheveu qui sont déficientes et d'activer la pousse du cheveu.
On peut expliquer cette action en considérant que la cystéine, qui prend naissance par ouverture du noyau de thiazolidine, exerce sur le système biochimique de la cellule qui élabore la kératine une action spécifique qui engendre par réaction une activation de la synthèse de la kératine.

Le zinc confère à cette molécule un pouvoir antioxydant. Cette propriété est importante car il a été démontré que l'une des causes d'apparition de pellicules provient de phénomènes d'oxydation qui peuvent être diminués en présence d'agents anti-oxydants.

La présente invention concerne également la préparation du L-thiazolidine-4 carboxylate de zinc.
Le nouveau composé, objet de l'invention, peut être obtenu, par exemple, de la manière suivante selon le schéma réactionnel :

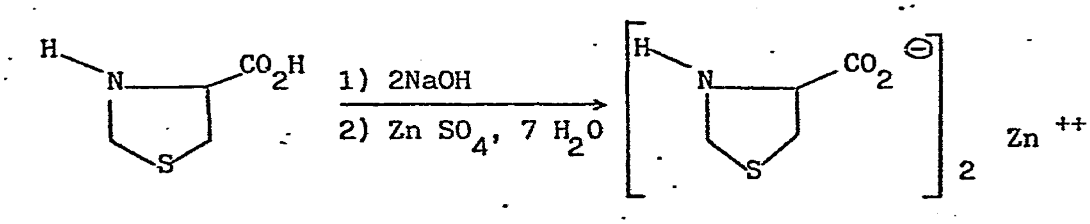


$+ Na_2 SO_4 + 9 H_2O$

Mode opératoire :

A une solution de 10,64 g (0,08 mole) d'acide L-thiazolidine-4 carboxylique dans 80 ml de soude N (0,08 mole), ajouter une solution de 11,48 g (0,04 mole) de sulfate de zinc heptahydrate dans 40 ml d'eau.
Il se forme aussitôt un abondant précipité blanc. Filtrer sur verre fritté, laver à l'eau et sécher à l'air, puis à l'étuve à 100°C jusqu'à poids constant. On obtient avec un rendement de 90 % le produit de formule :

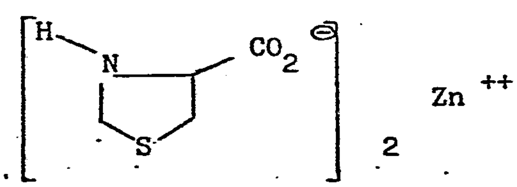

Formule brute : $C_8 H_{12} N_2 O_4 S_2 Zn$

Masse moléculaire : 329,69

Cristaux blancs

Point de décomposition : $\simeq$ 290°C

Contrôles : Lot MJL VIII 162

| | C | H | N | S | Zn |
|---|---|---|---|---|---|
| % théoriques | 29,14 | 3,67 | 8,50 | 19,45 | 19,82 |
| % trouvés | 29,11 | 3,67 | 8,30 | 19,65 | 19,56 |

Chromatographie sur plaque :

- support : gel de silice 60 F 254 Merck
- solvant : butanol - acide acétique - eau 6/2/2
- révélation : Ninhydrine
- Rf : 0,21 (acide L-thiazolidine-4 carboxylique).

Solubilité : Soluble dans l'eau à 1 %. Insoluble dans les solvants organiques.

EXPERIMENTATIONS :

1) Pouvoir antioxydant

La mesure du pouvoir antioxydant est effectuée selon la méthode décrite par CARNAT et POURRAT (Ann. Pharm. Fr. 1979, 37, n° 3-4, p. 119-124) en remplaçant l'huile de lin par de l'acide linoléique. L'oxydation de l'acide linoléique est suivie chaque jour par une mesure de l'indice de péroxyde Ip.

a) Indice de péroxydes

Dans un flacon contenant l'acide linoléique, on ajoute 15 ml de $CHCl_3$, 20 ml d'acide acétique RP puis 1 ml de solution aqueuse saturée de IK. On agite une minute et on laisse reposer le flacon 5 minutes à l'obscurité.

A près voir ajouté 100 ml d'eau, on dose l'iode libéré par du thiosulfate de sodium 0,01 N.

Les résultats obtenus sont regroupés dans le tableau suivant :

| Temps (h) \ Produits | Acide linoléique | L-thiazolidine carboxylate de Zn |
|---|---|---|
| 0 | 1,1 | 0,91 |
| 24 h | 47,30 | 12,7 |
| 48 h | 99,5 | 25.2 |

Les essais précédents démontrent l'excellente activité antioxydante du L-thiazolidine-4 carboxylate de zinc.

2) Pouvoir antilipasique

Le milieu d'incubation témoin ne contient que des acides gras estérifiés (tributyrate de glycérol).

L'ajout de lipase dans le milieu entraîne l'hydrolyse du tributyrate de glycérol.

L'incorporation de L-thiazolidine-4 carboxylate de zinc avant l'adjonction de lipase empêche la libération d'acide butyrique. Les résultats obtenus sont regroupés dans le tableau suivant :

| L-thiazolidine-4 carboxylate de Zn | % d'inhibition mesuré |
|---|---|
| 5 mg | 52 |
| 10 mg | 71,5 |

Le thiazolidine -4 carboxylate de Zn présente un fort pouvoir antilipasique associé à une bonne activité antioxydante, ce qui confère au produit un spectre d'activité intéressant dans le traitement des pellicules et des alopécies.

3) Tolérance locale

La tolérance a été recherchée sur 5 cobayes albinos adultes. L'application de produit trois fois par semaine pendant deux semaines sur le flanc préalablement rasé ne provoque aucune réaction.

4) Applications

Compte tenu de sa parfaite tolérance locale, de ses diverses propriétés (en particulier de son pouvoir antioxydant et antilipasique), le L-thiazolidine-4 carboxylate de zinc est utile/en cosmétologie et plus /dans les traitements dermatologiques, particulièrement pour :

- la cicatrisation et la régénération tissulaire, notamment après brûlures et coups de soleil,
- la protection vis à vis des radiations ionisantes,
- le ralentissement du vieillissement cutané,
- le traitement de l'acné et de la séborrhée (peau et cuir chevelu),

- le traitement des para et dyskératoses, ainsi que le soin des peaux sèches et des ichtyoses,
- l'atténuation des taches pigmentaires.

A titre d'exemples non limitatifs, quelques formulations sont données ci-après, dans lesquelles le L-thiazolidine-4 carboxylate de zinc peut être utilisé seul ou associé à d'autres composants doués de propriétés cosmétologiques dans les excipients appropriés, par exemple : antiseptiques et antifungiques, kératolytiques, rubéfiants, sébostatiques, anti-inflammatoires...

1) <u>Crème</u>

Mono et distéarate de PEG 300
Mono et distéarate de PEG 1 500
Acide stéarique
Huile de paraffine
L-thiazolidine-4 carboxylate de zinc
Conservateur
Eau

2) <u>Shampooing</u>

Alkyl éther sulfate de sodium
Ethanol amide d'acide gras de coprah
Bentonite modifié
Hydroxy propyl méthyl cellulose
L-thiazolidine-4 carboxylate de zinc
Parfum conservateur
Eau distillée

3) <u>Lait</u>

Mono et distéarate de PEG 300
Mono et distéarate de PEG 1 500
Huile de paraffine
L-thiazolidine-4 carboxylate de zinc
Conservateur, parfum
Eau distillée

4) Lotion.

Alcool à 95°

Eau d'hamamélis

L--thaizolidine-4 carboxylate de zinc

Parfum, conservateur

Hydroxy propyl cellulose

Eau distillée

REVENDICATIONS

1) A titre de produit industriel nouveau, le L-thiazolidine-4 carboxylate de zinc répondant à la formule :

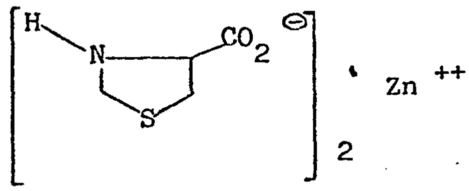

2) Procédé de préparation du L-thiazolidine-4 carboxylate de zinc, caractérisé en ce que l'on traite un sel de l'acide L-thiazolidine-4 carboxylique par une quantité stoechiométrique d'un sel de zinc dans un solvant.

3) Procédé selon la revendication 2, caractérisé en ce que le sel de l'acide L-thiazolidine carboxylique est le sel de sodium.

4) Procédé selon la revendication 2 et 3 caractérisé en ce que le sel de zinc est le sulfate de zinc.

5) Procédé selon les revendications 2 à 4 caractérisé en ce que le solvant utilisé est l'eau.

6) Application du L-thiazolidine-4 carboxylate de zinc en tant que substance active dans les compositions topiques dermatologiques et/ou cosmétologiques.

7) Les compositions topiques dermatologiques et cosmétologiques contenant, en tant que principe actif le L-thiazolidine-4 carboxylate de zinc seul ou associé à d'autres principes actifs au sein d'excipients compatibles avec la forme d'application.

8) Les compositions selon la revendication 7, caractérisées en ce qu'elles se présentent sous la forme de solutions, gels et crèmes dermiques destinés au traitement de diverses affections cutanées.

9) Les compositions selon la revendication 7, caractérisées en ce

qu'elles se présentent sous la forme de lotions ou de shampooings destinés au traitement de diverses affections du cuir chevelu.

Office européen des brevets

**RAPPORT DE RECHERCHE EUROPEENNE**

**0117176**
Numéro de la demande

EP 84 40 0141

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
| --- | --- | --- | --- |
| Y | US-A-3 243 346 (G. BECHMANN et al.) * En entier * | 1,6-9 | C 07 D 277/06<br>A 61 K 7/06<br>A 61 K 7/48<br>A 61 K 31/425 |
| Y | FR-M- 101 (SOGESPAR S.A.) * En entier * | 1,6-9 | |
| Y | EP-A-0 038 246 (PIERRE FABRE S.A.) * Revendications * | 6-9 | |
| Y | EP-A-0 034 385 (PROCTER & GAMBLE) * Revendications * | 6-9 | |
| | | | **DOMAINES TECHNIQUES RECHERCHES (Int. Cl. 3)** |
| | | | C 07 D 277/00<br>A 61 K 7/00 |

Le présent rapport de recherche a été établi pour toutes les revendications

| Lieu de la recherche | Date d'achèvement de la recherche | Examinateur |
| --- | --- | --- |
| LA HAYE | 14-05-1984 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82